# EUROPEAN PATENT APPLICATION

(11) **EP 3 804 619 A1**
(43) Date of publication of application: **14.04.2021**
(21) Application number: 19816089.7
(22) Date of filing: 04.06.2019
(51) Int. Cl.: A61B 5/1486, A61B 5/1473

(54) **HUMAN-BODY WEARABLE SENSOR DEVICE, SENSOR INSERTION DEVICE, AND SENSOR INSERTION METHOD**

(30) Priority: 06.06.2018 JP 2018108631; 06.06.2018 JP 2018108669
(71) Applicant: PHC Holdings Corporation, Tokyo 105-8433 (JP)
(72) Inventor: KOUGE, Masahiro, Ehime, 791-0395 (JP); IKETANI, Seiichiro, Ehime, 791-0395 (JP); SHIMIZU, Ryuji, Ehime, 791-0395 (JP); NISHIO, Akira, Ehime, 791-0395 (JP)
(74) Representative: Eisenführ Speiser
(86) International application number: PCT/JP2019/022185
(87) International publication number: WO 2019/235480

(57) **Abstract**

A wearable sensor device (1) comprises a sensor (5) at least part of which is inserted into a human body and which has a first connection terminal (7); a mount unit (6) on which at least part of the first connection terminal (7) of the sensor (5) is disposed and which is mounted on a skin of the human body; and an electronic unit (3) that is mounted to the mount unit (6) and has a second connection terminal (10) connected to the first connection terminal (7) of the sensor (5) via the mounting mechanism (8), and a control unit for acquiring signals from the sensor (5). The mounting mechanism (8) forms a first state where the mount unit (6) and the electronic unit (3) are mounted in a state in which the first connection terminal (7) and the second connection terminal (10) have yet to be connected, and a second state where the mount unit (6) and the electronic unit (3) are mounted in a state in which the first connection terminal and the second connection terminal have been connected.

## Description

### TECHNICAL FIELD

The present invention relates, for example, to a biosensor for measuring the concentration of glucose, amino acid, or the like in a living body, and a device for inserting this biosensor under the skin of a patient.

### BACKGROUND ART

A conventional wearable sensor device is configured to comprise a sensor that is inserted into a human body, a mount unit to which this sensor is mounted and which is mounted on the skin of the human body, and an electronic unit that is mounted to this mount unit, is electrically connected to the sensor, and has a control unit that calculates biometric information from signals inputted from the sensor.

The user attaches the mount unit to the skin, uses the sensor insertion device to insert the sensor into the human body through an opening in the mount unit, and then attaches the electronic unit to the mount unit, thereby electrically connecting the electronic unit and the sensor (see Patent Literature 1, for example).

Also, as an example of a sensor insertion device that inserts a sensor into a human body, a configuration of a puncture mechanism has been disclosed in which a needle is inserted into a human body in a state in which the sensor is enclosed in the needle, and then just the needle is pulled out by spring force (see Patent Literature 2, for example).

Also, there is a configuration in which a puncture mechanism for inserting and withdrawing a needle is constituted by a link mechanism that is linked to a trigger button (see Patent Literature 3, for example).

Also, a configuration has been disclosed in which a coil spring is used for a mechanism for inserting and withdrawing a needle (Patent Literature 4).

### CITATION LIST

### PATENT LITERATURE

Patent Literature 1: U.S. Patent No. 8,801,611
Patent Literature 2: U.S. Patent No. 9,402,544
Patent Literature 3: U.S. Patent Application Publication No. 2013/0267811
Patent Literature 4: WO 2017/187943

### SUMMARY

### TECHNICAL PROBLEM

A problem with the above-mentioned conventional wearable sensor device was that it was difficult to be attached to the human body.

That is, with a conventional wearable sensor device, the user inserted the sensor into the body, and then attached the electronic unit to the mount unit, thereby electrically connecting the electronic unit and the sensor. If the sensor was attached at a location that could not be seen (such as on the outside of the upper arm), it was difficult to visually check the mount unit and the sensor, so the electronic unit had to be attached to the mount unit and the sensor by feel.

Also, even when the electronic unit was attached in a visible location, the relatively small electronic unit had to be held in the fingers and attached to the mount unit and the sensor, so it was difficult to mount the wearable sensor device on the body.

In view of this, it is an object of the present invention to facilitate the mounting of a wearable sensor device on a human body.

### SOLUTION TO PROBLEM

To achieve this object, the wearable sensor device of the present invention comprises a sensor at least part of which is inserted into a human body, and which has a first connection terminal; a mount unit on which at least part of the first connection terminal of the sensor is disposed and which is mounted on the skin of a human body; an electronic unit that has a second connection terminal connected to the first connection terminal of the sensor, and a control unit for acquiring signals from the sensor; and a mounting mechanism that links the electronic unit to the mount unit in a rotatable state and mounts the electronic unit to the mount unit. The mounting mechanism forms a first state where the mount unit and the electronic unit are mounted in a state where the first connection terminal and the second connection terminal have yet to be connected, and a second state where the mount unit and the electronic unit are mounted in a state where the first connection terminal and the second connection terminal have been connected.

### ADVANTAGEOUS EFFECTS

With to the configuration of the present invention, after the sensor has been inserted into the human body, the electronic unit goes from the first state to the second state and is mounted to the mount unit via the mounting mechanism, which allows the wearable sensor device to be easily attached to the body.

### BRIEF DESCRIPTION OF DRAWINGS

FIG. 1 is a usage diagram of a continuous glucose monitoring (CGM) device in an embodiment of the present invention;
FIG. 2 is a usage diagram of the continuous glucose monitoring (CGM) device in FIG. 1;
FIG. 3a is a top view of the continuous glucose monitoring (CGM) device in FIG. 1, and FIG. 3b is an oblique view thereof;
FIG. 4a is an oblique view of the continuous glucose monitoring (CGM) device in FIG. 1, FIG. 4b is an oblique view thereof, and FIG. 4c is an oblique view thereof;
FIG. 5a is a top view of the continuous glucose monitoring (CGM) device in FIG. 1, and FIG. 5b is an oblique view thereof;
FIG. 6a is an oblique view of the continuous glucose monitoring (CGM) device in FIG. 1, FIG. 6b is an oblique view thereof, and FIG. 6c an oblique view thereof;
FIG. 7a is a top view of the continuous glucose monitoring (CGM) device in FIG. 1, and FIG. 7b is an oblique view thereof;
FIG. 8a is an oblique view of the continuous glucose monitoring (CGM) device in FIG. 1, FIG. 8b is an oblique view thereof, and FIG. 8c is an oblique view thereof;
FIG. 9 is a functional block diagram of the main parts of the continuous glucose monitoring (CGM) device in FIG. 1;
FIG. 10 is a usage diagram of the sensor insertion device in an embodiment of the present invention;
FIG. 11 is a usage diagram of the sensor insertion device in an embodiment of the present invention;
FIG. 12 is a usage diagram of the sensor insertion device in an embodiment of the present invention;
FIG. 13A is an oblique view of a sensor insertion device, and FIG. 13b is an oblique view thereof;
FIGS. 14a to 14d are transition diagrams of the sensor insertion device in an embodiment of the present invention;
FIGS. 15a to 15d are transition diagrams of the sensor insertion device in another embodiment of the present invention;
FIG. 16 is a flowchart of the sensor insertion method for a continuous glucose monitoring (CGM) device in an embodiment of the present invention; and
FIG. 17a is a detail view of the continuous glucose monitoring (CGM) device in FIG. 1, and FIG. 17b is a detail view of the hinge portion in FIG. 17a.

### DESCRIPTION OF EMBODIMENTS

### Embodiment 1

### Configuration of Continuous glucose monitoring (CGM) Device

As an example of a continuous glucose monitoring (CGM) system according to an embodiment of the present invention, a case of application to a glucose sensor for measuring glucose will now be described with reference to the appended drawings.

A continuous glucose monitoring (CGM) system continuously measures blood glucose levels in diabetic patients.

In the following description, "lower" means the puncture side (the side from which the needle protrudes) in the puncture direction in which the patient is punctured, and "upper" means the opposite side from the puncture side (the puncture knob side). General Description of Wearable Sensor Device 1

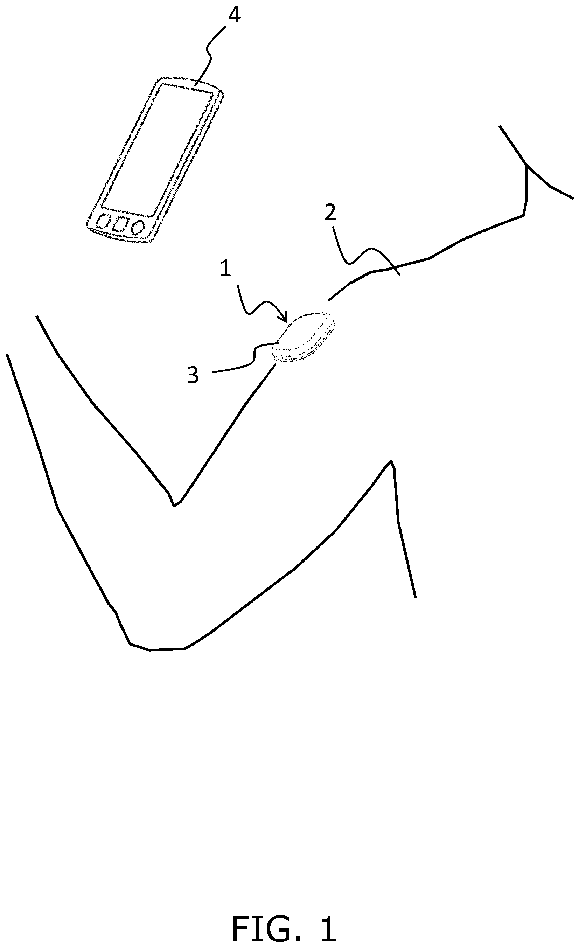

FIG. 1 shows a wearable sensor device 1 of a continuous glucose monitoring (CGM) system in this embodiment.

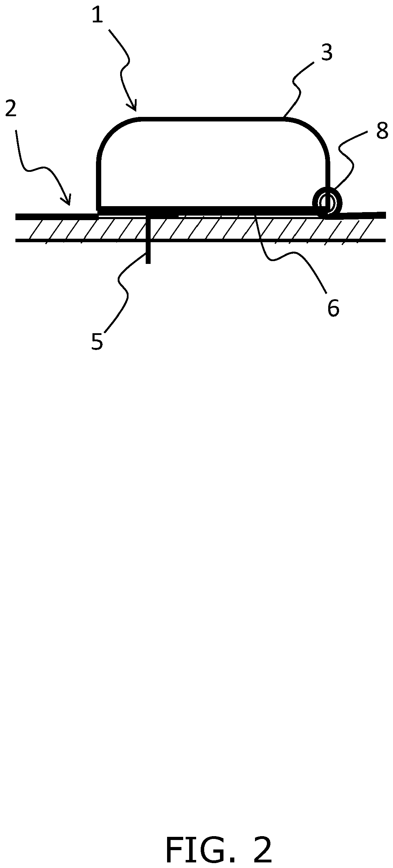

With the wearable sensor device 1 of the continuous glucose monitoring (CGM) system, a sensor 5 (see FIG. 2, etc.) is left under the skin of an upper arm 2 of a diabetic patient, and this sensor 5 is used to continuously monitor the glucose concentration in the interstitial fluid of the subcutaneous tissue.

The wearable sensor device 1 in this embodiment converts the glucose concentration into a current value, and transmits this calculated value. With the wearable sensor device 1, for example, the current value is measured once every minute, the arithmetic mean for a specific number of measurements is found, and that value is recorded in a memory. Then, the wearable sensor device 1 stores the five-minute value of the glucose concentration by storing the value of the arithmetic mean of the values for five samples acquired once every minute in the memory, for example.

As shown in FIG. 1, the wearable sensor device 1 wirelessly transmits and receives to and from a measurement device 4 by means of a substantially oblong electronic unit 3.

The wearable sensor device 1 stores in a memory the current value received by the electronic unit 3 from the sensor 5, or a glucose concentration value calculated on the basis of that current value, and wirelessly transmits the value stored in the memory to the measurement device 4. The value that is transmitted may be a current value, or may be a value converted into glucose concentration. The measurement device 4 calculates the glucose concentration from the value read by the sensor 5, displays the calculation result along with time information, and stores the time information and the glucose concentration in the memory inside the measurement device 4.

Continuously carrying out this glucose level measurement for about 3 to 14 days makes it possible to ascertain any fluctuation in the glucose level of a diabetic patient over the course of 24 hours. This means it is possible to perform a more appropriate treatment according to the symptoms of each diabetic patient on the basis of the fluctuation in blood glucose level.

Furthermore, the amount and timing of insulin administration to the patient can be calculated using this blood glucose level fluctuation information. An insulin pump administers the proper amount of insulin while monitoring the blood glucose level of the patient in real time while being connected wirelessly to the wearable sensor device 1 (and/or the measurement device 4). The system therefore functions as an artificial pancreas, so blood glucose can be kept at the ideal level.

FIG. 2 is a cross section of a portion of an upper arm 2 of a diabetic patient to which the wearable sensor device 1 has been attached.

With the wearable sensor device 1, the sensor 5 protrudes from the lower surface side that is in contact with the upper arm 2. The sensor 5 is placed under the skin when the wearable sensor device 1 is attached to the body.

The distal end portion of the sensor 5 is needle-shaped or rod-shaped to make it easier to insert this portion under the skin of a diabetic patient, and is about 1 cm in length. The distal end portion of the sensor 5 is covered with a protective film through which glucose (the test substance) permeates and is absorbed and which causes the glucose to react with an enzyme to produce a measurement substance, and a reaction layer that is constituted by an enzyme layer, a mediator layer, or the like.

An electrode for electrochemically measuring glucose is provided under the reaction layer. The glucose concentration in the interstitial fluid of the subcutaneous tissue can be observed (and/or measured/sensed) by leaving the electrode in the interstitial fluid in a state in which this distal end portion has been inserted under the skin.

With a subcutaneous indwelling glucose sensor such as this that measures glucose in subcutaneous tissue, there may be a time lag between the blood glucose level and the measurement result. This means that correction may be necessary using a measurement value from a glucose sensor for the self-monitoring of blood glucose (SMBG).

When the wearable sensor device 1 is mounted on the body, the mount unit 6 is disposed on the skin. The sensor 5 is attached to the mount unit 6 via a connection terminal. The electronic unit 3 is then mounted on the mount unit 6. The electronic unit 3 and the sensor 5 are connected via the mount unit 6.

### Configuration of Wearable Sensor Device 1

The configuration of the wearable sensor device 1 according to an embodiment of the present invention will now be described.

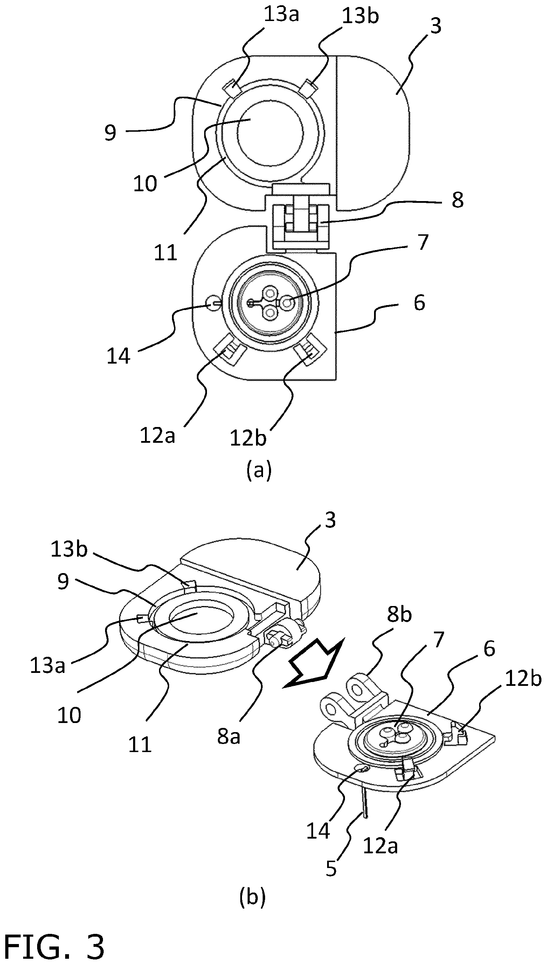

FIG. 3a shows a top view of the wearable sensor device 1, and FIG. 3b shows an oblique view thereof.

As shown in FIGS. 3a and 3b, the wearable sensor device 1 comprises the sensor 5, the mount unit 6, the electronic unit 3, and a hinge mechanism (mounting mechanism) 8.

At least a part of the sensor 5 is inserted into the patient's body, and the sensor 5 has a sensor connection terminal (first connection terminal) 7.

The mount unit 6 has at least a part of the sensor connection terminal 7 of the sensor 5 disposed thereon, and is mounted on the skin of the patient's body.

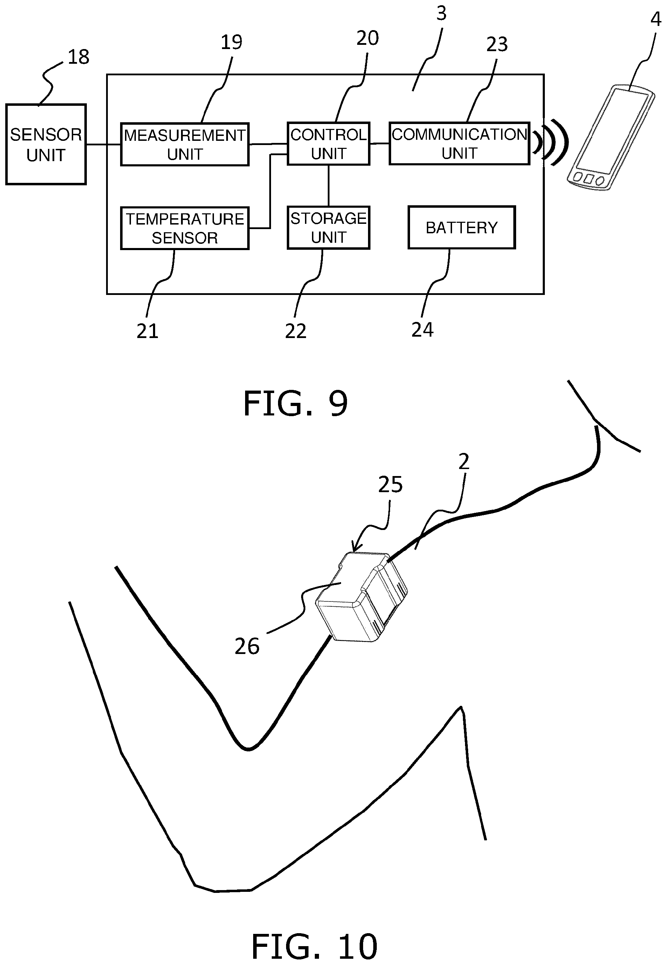

The electronic unit 3 has a second connection terminal 10 that is connected to the sensor connection terminal 7 of the sensor 5, and a control unit 20 (see FIG. 9) that acquires a signal from the sensor 5.

The hinge mechanism 8 allows the electronic unit 3 to rotate with respect to the mount unit 6 to switch between a contact state in which the electronic unit 3 is disposed opposite the mount unit 6 such that the surfaces thereof are close to each other, and a non-contact state. The hinge mechanism 8 forms a first state where the mount unit 6 and the electronic unit 3 are mounted in a state in which the sensor connection terminal 7 and the second connection terminal 10 have yet to be connected, and a second state where the mount unit 6 and the electronic unit 3 are mounted in a state in which the sensor connection terminal 7 and the second connection terminal 10 have been connected.

The hinge mechanism 8 is provided so as to link the ends of the electronic unit 3 and the mount unit 6 as shown in FIG. 3b. Furthermore, the hinge mechanism 8 detachably links the electronic unit 3 and the mount unit 6.

In a state in which the components have been disconnected by the hinge mechanism 8, the hinge mechanism 8 is separated into a hinge member 8a on the electronic unit 3 side and a hinge member 8b on the mount unit side. The hinge mechanism 8 is configured so that when a projection of the hinge member 8a is fitted into the an opening in the hinge member 8b, the electronic unit 3 is rotated with respect to the mount unit 6.

When both ends of the projection of the hinge member 8a are pivotally supported by the insertion opening in the hinge member 8b, the hinge member 8a rotates with respect to the hinge member 8b, with the projection serving as the axis of rotation. This results in the attachment of the electronic unit 3 to the mount unit 6 in an openable and closable state.

An insertion opening 9 into which a part of the sensor connection terminal 7 is fitted is provided on the bottom surface side of the electronic unit 3. The insertion opening 9 is provided with the second connection terminal 10 that connects to the sensor connection terminal 7.

An O-ring 11 is provided as a waterproof member around the periphery of the insertion opening 9. The O-ring 11 is disposed so as to be sandwiched between the electronic unit 3 and the mount unit 6 when the electronic unit 3 and the mount unit 6 are linked facing each other. As a result, the O-ring 11 functions as a waterproofing mechanism that prevents the intrusion of water or the like into the sensor connection terminal 7 and the second connection terminal 10.

The wearable sensor device 1 further comprises locking mechanisms 12 and 13 that fix the electronic unit 3 and the mount unit 6 in order to keep the electronic unit 3 and the mount unit 6 in a linked state (the second state). The locking mechanisms 12 and 13 have snap fits 12a and 12b provided on the mount unit 6 side, and insertion openings 13a and 13b provided on the electronic unit 3 side. In a state in which the electronic unit 3 and the mount unit 6 are linked to each other, the snap fit 12a and the insertion opening 13a, and the snap fit 12b and the insertion opening 13b are mated to each other. As a result, the linked state of the electronic unit 3 and the mount unit 6 is maintained.

The bottom surface of the mount unit 6 serves as an adhesive surface that is attached to the skin of the upper arm 2 or the like. An adhesive material such as a skin tape is provided on the bottom surface of the mount unit 6 to facilitate adhesion to the skin. When coupling the locking mechanisms 12 and 13, it is necessary to apply a downward force from above. Consequently, when a downward force is applied in coupling the locking mechanisms 12 and 13, this causes the bottom surface of the mount unit 6 to be pressed against the skin. As a result, the wearable sensor device 1 is more securely attached to the skin of the upper arm 2.

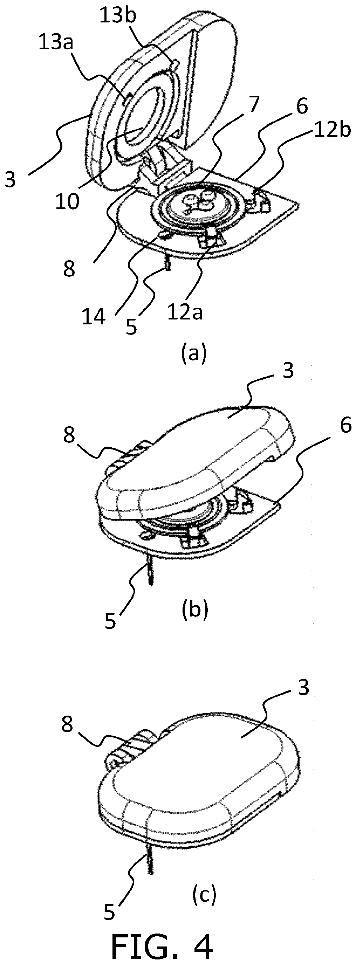

FIGS. 4a and 4b show the process by which the electronic unit 3 and the mount unit 6 are coupled to each other via the hinge mechanism 8.

The electronic unit 3 and the mount unit 6 are linked in a rotatable state via the hinge mechanism 8.

When the second connection terminal 10 of the electronic unit 3 and the sensor connection terminal 7 of the mount unit 6 have been connected, the two components are linked by rotating the electronic unit 3 around the protruding portion of the hinge member 8a of the hinge mechanism 8.

Here, the angle formed by the mount unit 6 and the electronic unit 3 around the protrusion of the hinge member 8a of the hinge mechanism 8 is defined as the rotation angle.

FIG. 4a shows the state when the rotation angle between the electronic unit 3 and the mount unit 6 is about 90 degrees (non-mounted state). FIG. 4b shows the state when the rotation angle of the electronic unit 3 and the mount unit 6 is about 30 degrees (non-mounted state). In these non-mounted states, the electronic unit 3 and the mount unit 6 are linked by the hinge mechanism 8, while the sensor connection terminal 7 and the second connection terminal 10 are not yet connected. This state is defined as the first state.

FIG. 4c shows the state when the rotation angle between the electronic unit 3 and the mount unit 6 has reached 0 degrees, and the electronic unit 3 and the mount unit 6 are completely coupled (mounted state). In this mounted state, the sensor connection terminal 7 and the second connection terminal 10 are connected. This state is defined as the second state.

In this way, the electronic unit 3 and the mount unit 6 are linked together by the hinge mechanism 8. The hinge mechanism 8 forms the first state where the mount unit 6 and the electronic unit 3 are mounted to each other in a state in which the sensor connection terminal 7 linked to the sensor 5 and the second connection terminal 10 provided on the electronic unit 3 side have yet to be connected.

The first state is a state in which the sensor connection terminal 7 and the second connection terminal 10 are not yet connected to each other, as shown in FIGS. 4a and 4b.

Furthermore, the hinge mechanism 8 forms the second state where the mount unit 6 and the electronic unit 3 are mounted in a state in which the sensor connection terminal 7 connected to the sensor 5 and the second connection terminal 10 provided on the electronic unit 3 side connected.

In the second state, as shown in FIG. 4c, the sensor connection terminal 7 and the second connection terminal 10 are connected to each other.

Thus, in this embodiment, the hinge mechanism 8 can form the first state where the mount unit 6 and the electronic unit 3 are attached to each other in a state in which the sensor connection terminal 7 and the second connection terminal 10 are not yet connected, and a second state where the mount unit 6 and the electronic unit 3 are attached to each other in a state in which the sensor connection terminal 7 and the second connection terminal 10 are connected, which allows the wearable sensor device 1 to be easily attached to the patient.

That is, after the sensor 5 has been inserted into the body, the electronic unit 3 is rotated with respect to the mount unit 6 via the hinge mechanism 8, and this simple operation switches from the first state to the second state, and attaches the electronic unit 3 to the mount unit 6, making it easy to attach the wearable sensor device 1 to the patient's body.

Consequently, even if the place where the sensor is to be attached cannot be seen (such as the outside of the upper arm part 2), the electronic unit 3 can be attached to the mount unit 6, and the sensor connection terminal 7 and the second connection terminal 10 can be connected, by the simple operation of rotating the electronic unit 3 via the hinge mechanism 8. As a result, the wearable sensor device 1 is easy to attach to the patient's body.

Since the mount unit 6 and the electronic unit 3 are rotatably linked together via the hinge mechanism 8, there is no need to grasp the relatively small electronic unit 3 to attach it to the mount unit 6 and the sensor 5. As a result, it is easy to attach the wearable sensor device 1 to the body.

Furthermore, the hinge mechanism 8 is provided with a spring or other such elastic body (not shown). An elastic body such as a spring biases the electronic unit 3 toward the mount unit 6.

Consequently, as shown in FIG. 4b, the electronic unit 3 of the wearable sensor device 1 rotates under spring force to a nearly closed state with respect to the mount unit 6 even when not being manually operated by the user.

As a result, the user can attach the electronic unit 3 to the mount unit 6 simply by lightly pressing on the electronic unit 3 from above with a finger, as shown in FIG. 4c. Here again, since a load is exerted on the wearable sensor device 1 from the upper side downward, the bottom surface of the mount unit 6 is pressed against the skin to which the device is to be mounted. As a result, the wearable sensor device 1 is more securely attached to the skin of the upper arm 2.

Also, since the electronic unit 3 covers the sensor connection terminal 7 disposed on the mount unit 6 as shown in FIG. 4b, this prevents the user from accidentally touching a finger to the second connection terminal 10 provided on the bottom surface of the electronic unit 3 and the sensor connection terminal 7.

The elastic body may be a torsion spring provided to the hinge mechanism, or may be a leaf spring.

Furthermore, a through-hole 14 through which passes a needle for puncturing the skin and inserting the sensor 5 into the body is provided above the sensor 5 of the mount unit 6.

In this embodiment, the electronic unit 3 is detachably attached to the mount unit 6 via the hinge mechanism 8 serving as a mounting mechanism.

Consequently, the electronic unit 3 can be attached to the mount unit 6 after the mount unit 6 and the sensor 5 attached to the mount unit 6 have been sterilized, so sterilization can be carried out in a state in which the electronic unit 3 has been separated.

Here, the reason for performing the sterilization with the electronic unit 3 separated is that the electronic unit 3 cannot be sterilized when electron beam sterilization is employed, for example, and therefore the electronic unit 3 must be separated from the member to be sterilized. Accordingly, because the electronic unit 3 is detachably mounted on the mount unit 6 via the hinge mechanism 8 serving as a mounting mechanism, sterilization can be performed with the electronic unit 3 in a separated state.

### Description of Electronic Unit 3

FIG. 9 shows a functional block diagram of the electronic unit 3.

The sensor unit 18 in FIG. 9 has the above-mentioned sensor 5 and sensor connection terminal 7.

With the electronic unit 3, the current value sensed by the sensor unit 18 and measured by a measurement unit 19 is transmitted to a control unit 20. In the control unit 20, the temperature near the sensor unit 18 is measured by a temperature sensor 21, the temperature is corrected, and the glucose concentration is calculated from the current value.

The control unit 20 repeatedly executes this calculation of the glucose concentration at predetermined sampling intervals. In this embodiment, this sampling interval is set to 1 minute, for example.

The control unit 20 then calculates an integrated average for the calculated glucose concentration at each predetermined recording interval, and records this result to the storage unit 22. In this embodiment, this recording interval is set to 5 minutes, for example. Consequently, the integrated average for 5 samples measured every 1 minute for 5 minutes is calculated, and the integrated average is recorded in the storage unit 22.

The control unit 20 transmits the value stored in the storage unit 22 to the measurement device 4 via the communication unit 23 according to an instruction from the measurement device 4.

The electronic unit 3 in this embodiment has a built-in battery 24 that supplies power to the measurement unit 19, the control unit 20, the temperature sensor 21, the communication unit 23, and so on. Usually, the electronic unit 3 is discarded at the point when the remaining battery level of the battery 24 becomes insufficient. The remaining battery level of the battery 24 is monitored through the measurement device 4.

When the sensor unit 18 is replaced, the measurement device 4 confirms whether or not the remaining charge of the battery 24 is sufficient to last until the next replacement of the sensor unit 18, with respect to the remaining charge of the battery 24 of the electronic unit 3. If the remaining charge is too low, the control unit 20 outputs an instruction message or audio telling the user to replace the electronic unit 3, and performs control so that the electronic unit 3 cannot be used.

In addition, in the configuration of the wearable sensor device 1 according to Embodiment 3 above, the electronic unit 3 is removably attached to the electronic unit housing 17. With this configuration, the battery (battery) 24 is located in a battery disposition unit provided to the electronic unit housing 17, rather than in the electronic unit 3, and supplies electric power to the electronic unit 3.

Consequently, the battery 24 does not have to be installed in the electronic unit 3, so the electronic unit 3 can be more compact.

Furthermore, when the sensor unit 18 is to be replaced, the electronic unit 3 is removed from the electronic unit housing 17, and the sensor unit 18, the mount unit 6, and the electronic unit housing 17 are discarded along with the battery 24. A new sensor unit 18, mount unit 6, electronic unit housing 17, and battery 24 are then installed, and the electronic unit housing 17 is attached.

This means that the electronic unit 3 can be used for an extended period of time, without its replacement depending on the remaining charge of the battery 24, so the cost of the product can be reduced.

The battery 24 may be disposed in the mount unit 6. In this case, since there is no need to install the battery 24 in the electronic unit 3, the electronic unit 3 can be more compact. Furthermore, when the sensor unit 18 is to be replaced, the electronic unit 3 is removed from the mount unit 6, and the sensor unit 18 and the mount unit 6 are discarded along with the battery 24. Installing a new sensor unit 18, mount unit 6, and battery 24 allows the electronic unit 3 to be used for an extended period of time, without its replacement depending on the remaining charge of the battery 24, so the cost of the product can be reduced.

### Usage Example of Mounting the Wearable Sensor Device 1 on a Body

FIGS. 10, 11, and 12 show examples of mounting the wearable sensor device 1 on a patient's body.

In FIG. 10, a sensor insertion device 25, in the interior of which is held the wearable sensor device 1 of a continuous glucose monitoring (CGM) system, is in contact with the upper arm 2 of a diabetic patient, and an upper case 26 is being pushed downward while held in a hand.

In this state, the sensor 5 of the wearable sensor device 1 has punctured the skin of the patient.

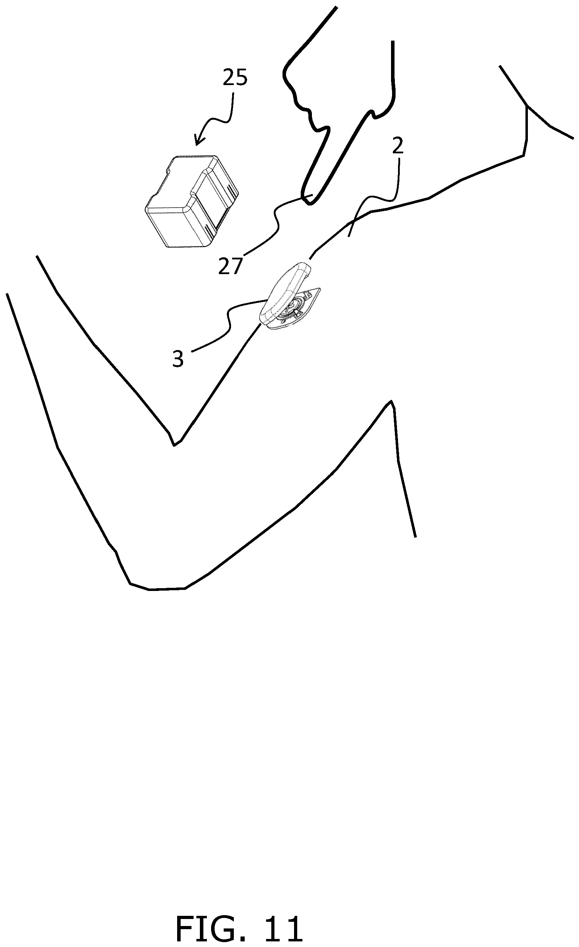

FIG. 11 shows a state in which the sensor insertion device 25 has been lifted upward from the state shown in FIG. 10. In this state, the sensor insertion device 25 has released the wearable sensor device 1. The electronic unit 3 of the wearable sensor device 1 is slightly open with respect to the mount unit 6.

The electronic unit 3 is linked to the mount unit 6 via the hinge mechanism 8 or 58 as described above. In a state in which the sensor insertion device 25 has released the wearable sensor device 1, the electronic unit 3 rotates and moves toward the mount unit 6 under its own weight. Alternatively, if there is a spring unit 16, such as with the hinge mechanism 58, the spring force of this spring unit 16 causes the electronic unit 3 to be substantially in contact with the mount unit 6 (closed).

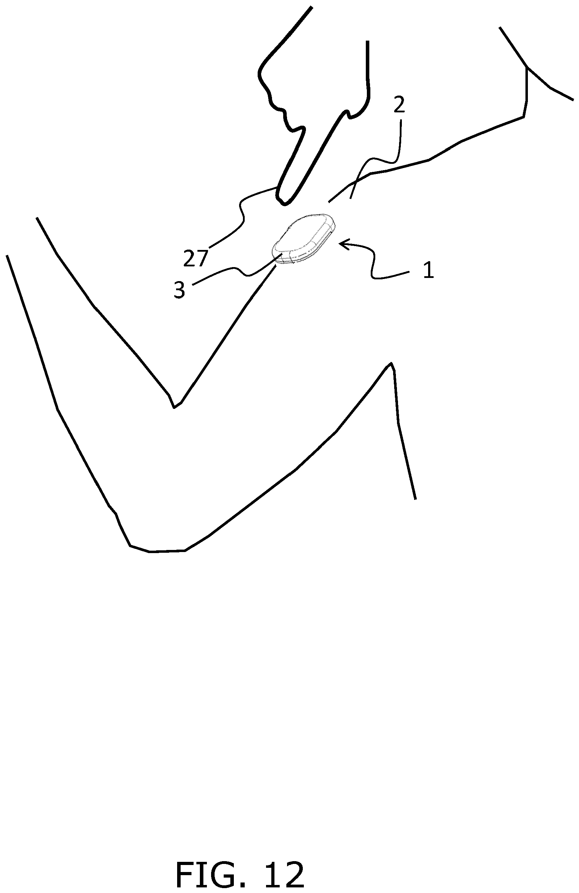

In FIG. 12, the user pushes the upper surface of the electronic unit 3 downward with the finger 27, causing the electronic unit 3 and the mount unit 6 to move close to each other and be linked in that state. At this point, the sensor connection terminal 7 of the sensor unit 18 mounted on the mount unit 6 is connected to the second connection terminal 10 of the electronic unit 3. The wearable sensor device 1 is electrically actuated, starts communicating with the measurement device 4, and starts measuring the glucose concentration of interstitial fluid under the patient's skin.

An adhesive material is provided on the bottom surface of the mount unit 6. As described above, when the user pushes the upper surface of the electronic unit 3 downward with the finger 27, the mount unit 6 is pressed against the surface of the skin of the upper arm 2. Therefore, the adhesive material on the bottom surface of the mount unit 6 is more securely bonded to the skin of the upper arm 2.

### Structure of Sensor Insertion Device 25

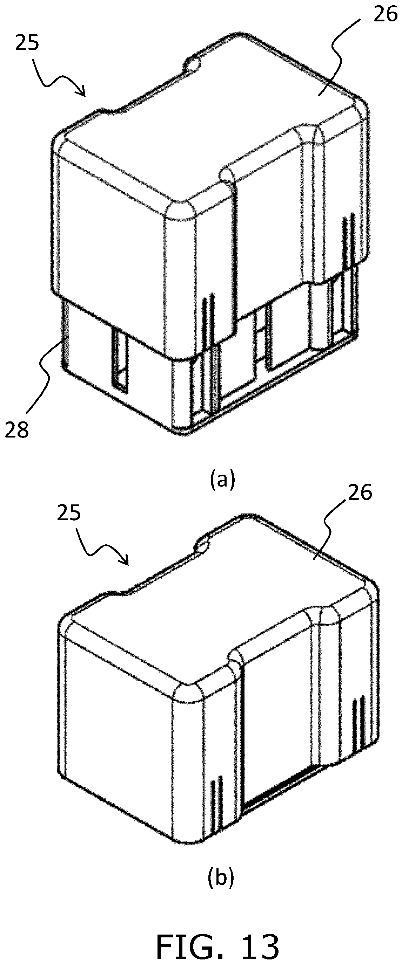

FIGS. 13a and 13b are external views of the sensor insertion device 25.

FIG. 13a shows the state before the upper case 26 is pushed downward, and FIG. 13b shows the state when the upper case 26 has been pushed downward.

As shown in FIG. 13a, the sensor insertion device 25 has, as a main body case, an upper case 26 and a lower case 28 that is configured to be vertically slidable with respect to the upper case 26.

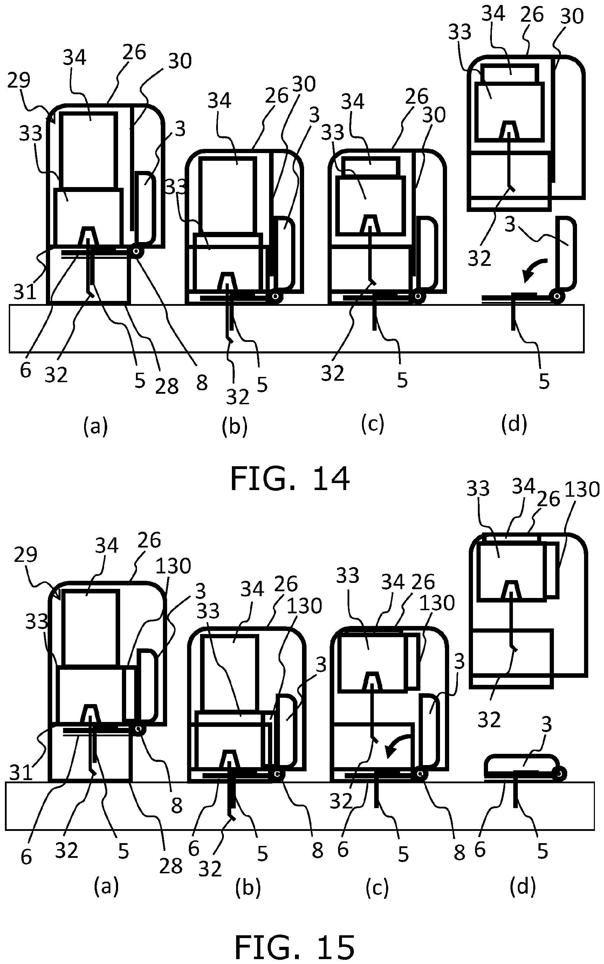

The internal configuration of the sensor insertion device 25 in this embodiment will now be described. FIGS. 14a to 14d show the internal state of the sensor insertion device 25 during sensor insertion.

As shown in FIG. 14a, the sensor insertion device 25 comprises, inside the main body case (the upper case 26 and the lower case 28), a sensor insertion mechanism 29 that inserts the sensor 5 into the body, a support unit 30 that supports the electronic unit 3 in the above-mentioned first state while the sensor 5 is being inserted into the body, and a holding unit 31 that holds the mount unit 6.

The sensor insertion mechanism 29 has a puncture needle 32, a puncture needle holder 33, and a drive unit 34 that drives the puncture needle holder 33 downward in the puncturing direction, and pulls up the puncture needle holder 33 after the puncture to withdraw the puncture needle 32 from the skin.

In the configuration of the sensor insertion mechanism 29, the springs described in the above-mentioned Patent Literature 2 and 4, for example, may be used as the drive unit 34. Also, the link mechanism described in Patent Literature 3 may be used as the drive unit 34. In this embodiment, the specific configuration of the drive unit 34 will not be described in order to keep the description from becoming too complicated.

The support unit 30 is provided as a wall surface formed so as to extend downward from the ceiling surface in the upper case 26, and in the state shown in FIGS. 14a to 14c, the electronic unit 3 is supported in a state in which the rotation angle is about 90 degrees.

Let us return to the description of FIGS. 14a to 14d.

FIG. 14a shows the state before the upper case 26 is pushed downward.

Inside the upper case 26, the mount unit 6 of the wearable sensor device 1 is held by the holding unit 31 in the lower part of the upper case 26.

The puncture needle 32 is in a state in which the sensor 5 is included therein.

The electronic unit 3 connected by the hinge mechanism 58 to the mount unit 6 is supported by the support unit 30. The electronic unit 3 is linked in a rotatable state to the mount unit 6 by the hinge mechanism 58. Therefore, the support unit 30 only needs to support the electronic unit 3 so as not to rotate.

FIG. 14b shows the state when the upper case 26 has been pushed downward. In this state, the puncture needle 32 is inserted under the skin with the sensor 5 included therein.

FIG. 14c shows the state when the puncture needle holder 33 has been pulled up by the drive unit 34. In this state, the sensor 5 is separated from the puncture needle 32 and is left under the skin.

FIG. 14d shows the state when the user has lifted up the sensor insertion device 25. In this state, since the support unit 30 is fixed inside the upper case 26, when the sensor insertion device 25 is lifted up, the support unit 30 is also lifted up and the support of the electronic unit 3 by the support unit 30 is released. That is, the electronic unit 3 is supported by the support unit 30 in the above-mentioned first state until the sensor 5 is inserted into the body. Once the sensor 5 has been inserted into the body, the support unit 30 releases the support of the electronic unit 3.

Consequently, it is possible to form a first state in which the mount unit 6 and the electronic unit 3 of the wearable sensor device 1 are mounted in a state where the sensor connection terminal 7 and the second connection terminal 10 have yet to be connected, and a second state in which the mount unit 6 and the electronic unit 3 are mounted in a state where the connection terminal 7 and the second connection terminal 10 are connected. As a result, the wearable sensor device 1 can be easily attached to the patient's body.

That is, with the configuration of this embodiment, after the sensor 5 is inserted into the body, the support of the electronic unit 3 by the support unit 30 is released, and the electronic unit 3 goes from the first state to the second state, so that the electronic unit 3 is mounted on the mount unit 6. As a result, the wearable sensor device 1 can be easily attached to the patient's body.

### Flowchart of Sensor Mounting Method

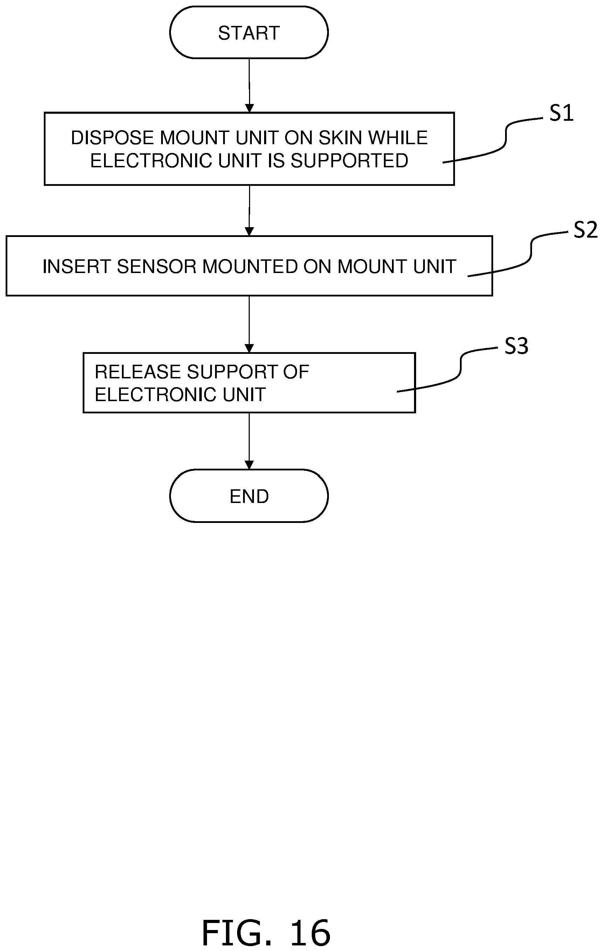

FIG. 16 is a flowchart of the sensor mounting method according to this embodiment.

The wearable sensor device 1 of this embodiment comprises the sensor 5, at least part of which is inserted into the patient's body, and which has the sensor connection terminal (first connection terminal) 7. At least part of the sensor connection terminal 7 of the sensor 5 is disposed on the mount unit 6 that is mounted on the skin of the patient. The electronic unit 3 has a second connection terminal 10 that is attached to the mount unit 6 via the hinge mechanism 8 of the attachment mechanism and is connected to the sensor connection terminal 7 of the sensor 5, and a control unit 20 (see FIG. 9) that calculates biometric information from the signal inputted from the sensor 5.

The hinge mechanism 8 forms a first state in which the mount unit 6 and the electronic unit 3 are attached to each other in a state where the sensor connection terminal 7 and the second connection terminal 10 have yet to be connected, and a second state in which the mount unit 6 and the electronic unit 3 are attached to each other in a state where the sensor connection terminal 7 and the connection terminal 10 are connected.

A sensor insertion method for inserting the sensor 5 into the body using this wearable sensor device 1 will now be described.

First, a step of placing the mount unit 6 on the skin (S1) is performed in the first state in which the sensor connection terminal 7 of the mount unit 6 and the second connection terminal 10 of the electronic unit 3 have yet to be connected, that is, in a state in which the electronic unit 3 is supported by the support unit 30.

Next, a step of inserting the sensor 5 mounted on the mount unit 6 into the patient's body is performed (S2).

After the sensor 5 has been inserted into the body, a step of creating a second state in which the sensor connection terminal 7 of the mount unit 6 and the second connection terminal 10 of the electronic unit 3 are connected is performed (S3).

This forms a first state in which the mount unit 6 and the electronic unit 3 are attached to each other in a state where the sensor connection terminal 7 and the second connection terminal 10 have yet to be connected, and a second state in which the mount unit 6 and the electronic unit 3 are attached to each other in a state where the sensor connection terminal 7 and the second connection terminal 10 are connected. As a result, the wearable sensor device 1 can be easily attached to the body.

### Embodiment 2

The configuration of the wearable sensor device 1 according to another embodiment of the present invention will now be described.

This embodiment differs from Embodiment 1 given above in that the configuration of the hinge mechanism 58 serving as a mounting mechanism that links the electronic unit 3 and the mount unit 6 together is different from the configuration of the hinge mechanism 8 discussed above. The rest of the configuration is the same as that in Embodiment 1, and those components will be numbered the same and will not be described in detail.

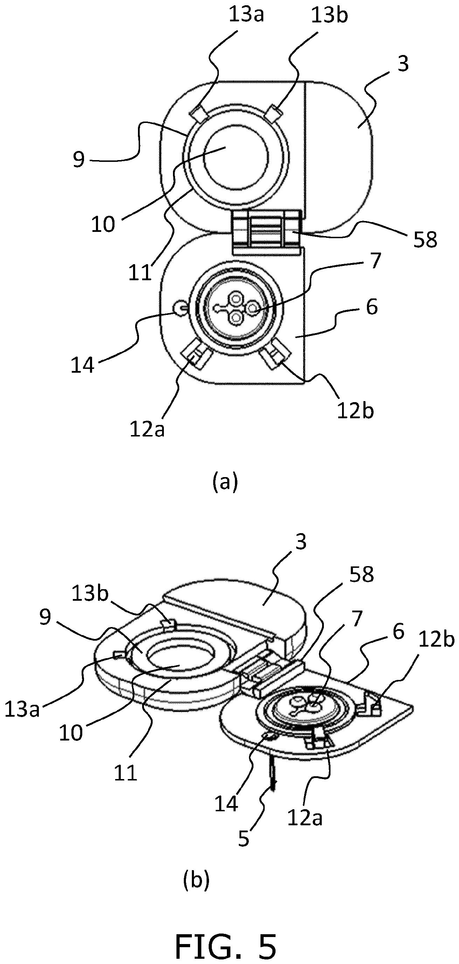

FIG. 5a is a top view of the wearable sensor device 1, and FIG. 5b is an oblique view thereof.

The wearable sensor device 1 comprises the electronic unit 3, the sensor 5, the mount unit 6, and the hinge mechanism 58.

At least part of the sensor 5 is inserted into the patient's body, and the sensor 5 has a sensor connection terminal (first connection terminal) 7.

The mount unit 6 has at least a part of the sensor connection terminal 7 of the sensor 5 disposed thereon, and is attached to the skin of the patient's body.

The electronic unit 3 has a second connection terminal 10 that is attached to the mount unit 6 via the hinge mechanism 58 (serving as an attachment mechanism) and is connected to the sensor connection terminal 7, and a control unit 20 (see FIG. 9) that calculates biometric information from the signal inputted from the sensor 5.

The hinge mechanism 58 (mounting mechanism) forms a first state in which the mount unit 6 and the electronic unit 3 are mounted to each other in a state where the sensor connection terminal 7 and the second connection terminal 10 have yet to be connected, and a second state in which the mount unit 6 and the electronic unit 3 are attached to each other in a state where the sensor connection terminal 7 and the second connection terminal 10 have are connected.

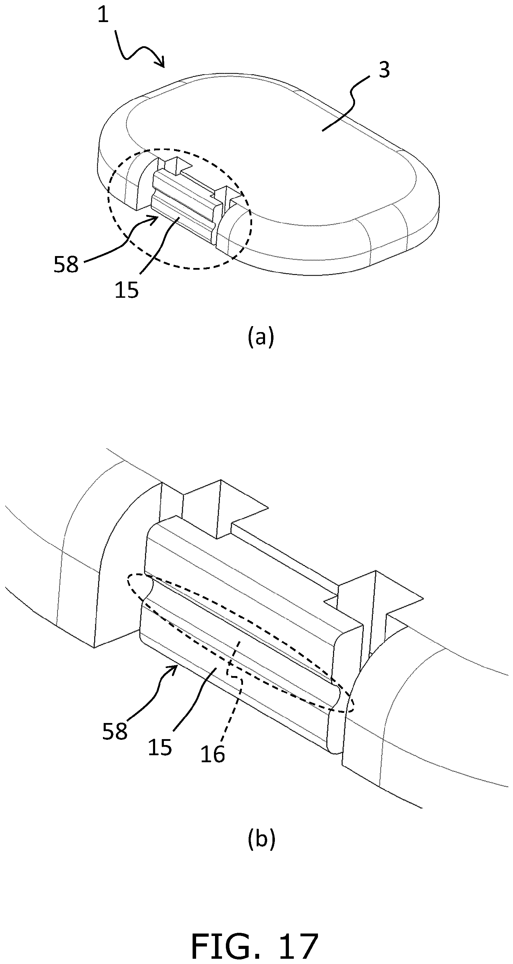

As shown in FIGS. 5a and 5b, the hinge mechanism 58 is configured such that the electronic unit 3 and the mount unit 6 are linked together. The hinge mechanism 58 has a hinge portion 15 and a spring unit (elastic body) 16, as shown in FIGS. 17a and 17b.

As shown in FIG. 17a, the hinge portion 15 is formed as a portion that links the electronic unit 3 and the mount unit 6 together in the hinge mechanism 58. The hinge portion 15 is molded integrally with the electronic unit 3 side from a thermoplastic resin such as polypropylene (PP). That is, the hinge portion 15 is integrally molded as a part of the electronic unit 3.

As shown in FIG. 17b, the spring unit 16 is a thin-walled portion of the hinge portion 15, and is formed so as to be bendable.

Here, the hinge portion 15 is formed at an angle at which the electronic unit 3 will be closed with respect to the mount unit 6.

Consequently, the thin-walled spring unit 16 of the hinge portion 15 exerts a biasing force that biases the electronic unit 3 in the closing direction when the electronic unit 3 has been opened with respect to the mount unit 6.

The bottom surface of the electronic unit 3 is provided with an insertion opening 9 into which part of the sensor connection terminal 7 fits. The second connection terminal 10 that is connected to the sensor connection terminal 7 is provided to the insertion opening 9.

An O-ring 11 is provided as a waterproof member around the periphery of the insertion opening 9. The O-ring 11 is disposed so as to be sandwiched between the electronic unit 3 and the mount unit 6 when the electronic unit 3 and the mount unit 6 are linked together. As a result, the O-ring 11 functions as a waterproofing mechanism that prevents water and the like from infiltrating the sensor connection terminal 7 and the second connection terminal 10.

The wearable sensor device 1 further comprises locking mechanisms 12 and 13 for maintaining a state in which the electronic unit 3 and the mount unit 6 are linked. The locking mechanisms 12 and 13 have snap fits 12a and 12b provided on the mount unit 6 side, and insertion openings 13a and 13b provided on the electronic unit 3 side. In a state in which the electronic unit 3 and the mount unit 6 are linked together, the snap fit 12a and the insertion opening 13a, and the snap fit 12b and the insertion opening 13b are fitted together. As a result, the linked state of the electronic unit 3 and the mount unit 6 is maintained.

The bottom surface of the mount unit 6 serves as an adhesive surface that is attached to the skin of the upper arm 2 or the like. An adhesive material such as skin tape is provided on the bottom surface of the mount unit 6 to facilitate bonding to the skin. In linking the locking mechanisms 12 and 13, it is necessary to apply a downward force from above. Therefore, when a downward force is exerted in the linking of the locking mechanisms 12 and 13, the bottom surface of the mount unit 6 is pressed against the skin where the device is to be attached. As a result, the wearable sensor device 1 is more securely attached to the skin of the upper arm 2.

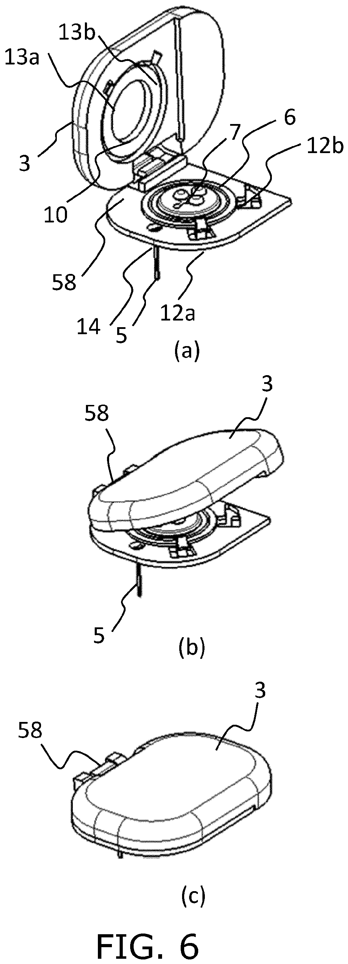

FIGS. 6a to 6c show the process by which the electronic unit 3 and the mount unit 6 are coupled to each other via the hinge mechanism 58.

The electronic unit 3 and the mount unit 6 are rotatably connected by the hinge mechanism 58.

When the electronic unit 3 and the mount unit 6 are linked together, the electronic unit 3 is coupled so as to rotate with the hinge mechanism 58 as its fulcrum.

Here, the angle formed by the mount unit 6 and the electronic unit 3 around the hinge mechanism 58 is defined as the rotation angle.

FIG. 6a shows the state (non-attached state) when the rotation angle of the electronic unit 3 and the mount unit 6 is about 90 degrees. FIG. 6b shows the state (non-attached state) when the rotation angle of the electronic unit 3 and the mount unit 6 is about 30 degrees. In these non-contact states, the electronic unit 3 and the mount unit 6 are attached to each other via the hinge mechanism 58, and the sensor connection terminal 7 and the second connection terminal 10 are not yet connected. This state is defined as the first state.

FIG. 6c shows the state (attached state) when the rotation angle of the electronic unit 3 and the mount unit 6 is zero degrees, and the electronic unit 3 and the mount unit 6 are completely coupled. This is a state in which the sensor connection terminal 7 and the second connection terminal 10 are connected. This state is defined as the second state.

Thus, the electronic unit 3 and the mount unit 6 are linked to each other by the hinge mechanism 58. The hinge mechanism 58 forms the first state in which the mount unit 6 and the electronic unit 3 are attached to each other in a state where the sensor connection terminal 7 connected to the sensor 5 and the second connection terminal 10 provided on the electronic unit 3 side have yet to be connected.

The first state is a state where the sensor connection terminal 7 and the second connection terminal 10 have yet to be connected to each other, as shown in FIGS. 6a and 6b.

Furthermore, the hinge mechanism 58 forms a second state in which the mount unit 6 and the electronic unit 3 are attached in a state where the sensor connection terminal 7 connected to the sensor 5 and the second connection terminal 10 provided on the electronic unit 3 side are connected.

This second state, as shown in FIG. 6c, is a state in which the sensor connection terminal 7 and the second connection terminal 10 are connected to each other.

Thus, in this embodiment, because the hinge mechanism 58 forms the first state in which the mount unit 6 and the electronic unit 3 are attached to each other in a state where the sensor connection terminal 7 and the second connection terminal 10 have yet to be connected, and the second state in which the mount unit 6 and the electronic unit 3 are attached to each other in a state where the sensor connection terminal 7 and the second connection terminal 10 are connected to each other, the wearable sensor device 1 can be easily attached to the body.

That is, after the sensor 5 has been inserted into the body, the electronic unit 3 is rotated with respect to the mount unit 6 via the hinge mechanism 58, and this simple operation causes a transition from the first state to the second state and mounts the electronic unit 3 on the mount unit 6, so the wearable sensor device 1 can be easily attached to the body.

Consequently, even if the sensor is attached at a location there cannot be seen (such as on the outside of the upper arm 2), the electronic unit 3 can be mounted on the mount unit 6, and the sensor connection terminal 7 and the second connection terminal 10 can be connected, by the simple operation of rotating the electronic unit 3 via the hinge mechanism 58. As a result, the wearable sensor device 1 can be easily attached to the body.

Also, since the mount unit 6 and the electronic unit 3 are rotatably linked via the hinge mechanism 58, there is no need to grasp the relatively small electronic unit to attach it to the mount unit 6 and the sensor 5. As a result, the wearable sensor device 1 can be easily attached to the body.

Furthermore, the hinge mechanism 58 is provided with an elastic body as described above. This elastic body is configured to bias the electronic unit 3 in the direction of mounting on the mount unit 6.

In this embodiment, the elastic body is constituted by the spring unit 16 provided to the hinge mechanism 58. The spring unit 16 is formed integrally with the hinge portion 15 using a resin or other such material. Therefore, the hinge mechanism 58 having an elastic body can be formed without increasing the number of parts.

Consequently, as shown in FIG. 6b, the electronic unit 3 of the wearable sensor device 1 is substantially closed with respect to the mount unit 6 even when the user is not operating it by hand.

As a result, the user can mount the electronic unit 3 to the mount unit 6 as shown in FIG. 6c simply by lighting pressing on it with a finger from above. Here again, since a load is exerted on the wearable sensor device 1 downward from above, the bottom surface of the mount unit 6 is pressed against the skin where the device is to be mounted. Therefore, the wearable sensor device 1 is more securely attached to the skin of the upper arm 2.

Also, since the electronic unit 3 covers the sensor connection terminal 7 disposed on the mount unit 6 as shown in FIG. 6b, this prevents the user from accidentally touching a finger to the second connection terminal 10 provided to the bottom surface of the electronic unit 3, or to the sensor connection terminal 7.

The elastic body may be a torsion spring or a leaf spring linked to the hinge mechanism.

Furthermore, a through-hole 14 through which passes a needle for puncturing the skin and inserting the sensor 5 into the body is provided above the sensor 5 of the mount unit 6.

### Embodiment 3

The configuration of the wearable sensor device 1 according to yet another embodiment of the present invention will be now described.

The wearable sensor device 1 of this embodiment differs from Embodiments 1 and 2 above in that it comprises an electronic unit housing 17 in which the electronic unit 3 is mounted. The rest of the configuration is the same as that in Embodiments 1 and 2, and those components will be numbered the same and will not be described in detail.

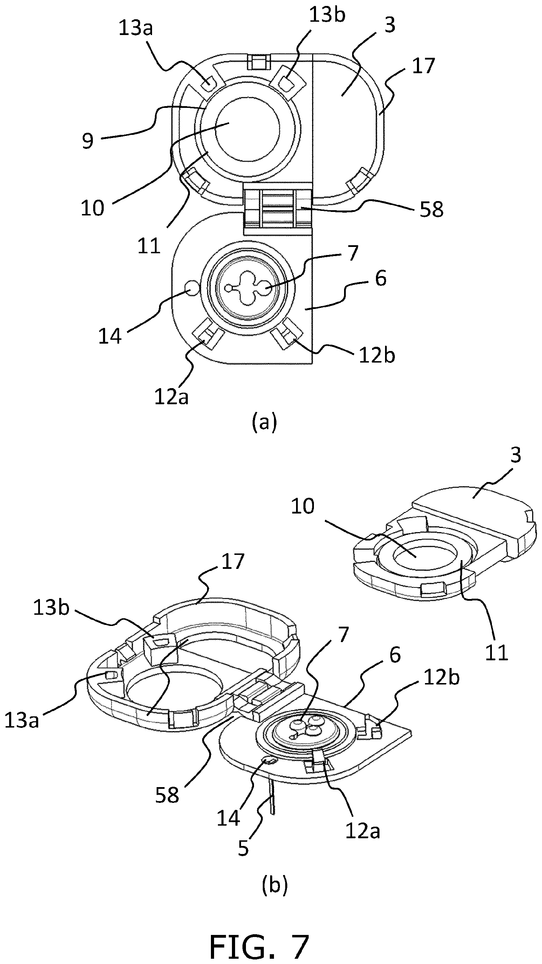

FIG. 7a is a top view of the wearable sensor device 1, and FIG. 7b is an oblique view thereof.

The wearable sensor device 1 comprises the electronic unit 3, the sensor 5, the electronic unit housing 17, and the hinge mechanism 58.

At least part of the sensor 5 is inserted into the patient's body, and the sensor 5 has a sensor connection terminal (first connection terminal) 7.

The mount unit 6 has at least a part of the sensor connection terminal 7 of the sensor 5 disposed thereon, and is attached to the skin of the patient's body.

The electronic unit housing 17 is mounted on the mount unit 6 via the hinge mechanism 58 serving as a mounting mechanism.

The electronic unit 3 is removably attached to the electronic unit housing 17, and has the second connection terminal 10 connected to the sensor connection terminal 7, and a control unit that calculates biometric information from signals inputted from the sensor 5 (see FIG. 9).

The hinge mechanism 58 (mounting mechanism) forms a first state in which the mount unit 6 and the electronic unit housing 17 are attached to each other in a state where the sensor connection terminal 7 and the second connection terminal 10 have yet to be connected, and a second state in which the mount unit 6 and the electronic unit housing 17 are attached to each other in a state where the second connection terminal 10 and the second connection terminal 10 are connected to each other.

As shown in FIGS. 7a and 7b, the hinge mechanism 58 is configured to link the electronic unit housing 17 and the mount unit 6 together. The hinge mechanism 58 has the hinge portion 15 and the spring unit 16. The configuration of the hinge portion 15 and the spring unit 16 is as described above with reference to FIGS. 17a and 17b.

An insertion opening 9 into which a part of the sensor connection terminal 7 is fitted is provided on the bottom surface side of the electronic unit 3. The insertion opening 9 is provided with the second connection terminal 10 that connects to the sensor connection terminal 7.

An O-ring 11 is provided as a waterproof member around the periphery of the insertion opening 9. The O-ring 11 is disposed so as to be sandwiched between the electronic unit 3 and the mount unit 6 when the electronic unit 3 and the mount unit 6 are linked together. As a result, the O-ring 11 functions as a waterproofing mechanism that prevents the intrusion of water or the like into the sensor connection terminal 7 and the second connection terminal 10.

The wearable sensor device 1 further comprises locking mechanisms 12 and 13 that fix the electronic unit 3 and the mount unit 6 in order to keep the electronic unit 3 and the mount unit 6 in a linked state (the second state). The locking mechanisms 12 and 13 have snap fits 12a and 12b provided on the mount unit 6 side, and insertion openings 13a and 13b provided on the electronic unit 3 side. In a state in which the electronic unit 3 and the mount unit 6 are linked to each other, the snap fit 12a and the insertion opening 13a, and the snap fit 12b and the insertion opening 13b are mated to each other. As a result, the linked state of the electronic unit 3 and the mount unit 6 is maintained.

The bottom surface of the mount unit 6 serves as an adhesive surface that is attached to the skin of the upper arm 2 or the like. An adhesive material such as a skin tape is provided on the bottom surface of the mount unit 6 to facilitate adhesion to the skin. When coupling the locking mechanisms 12 and 13, it is necessary to apply a downward force from above. Consequently, when a downward force is applied in coupling the locking mechanisms 12 and 13, this causes the bottom surface of the mount unit 6 to be pressed against the skin. As a result, the wearable sensor device 1 is more securely attached to the skin of the upper arm 2.

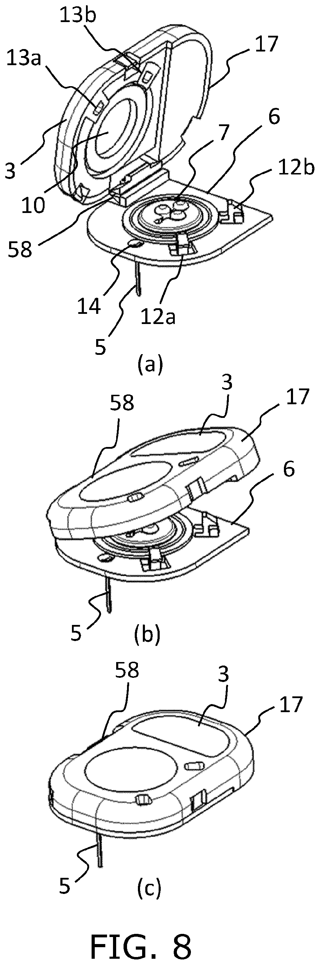

FIGS. 8a to 8c show the process in which the electronic unit housing 17 and the mount unit 6 are coupled to each other via the hinge mechanism 58. At this point, the electronic unit 3 is mounted in the electronic unit housing 17.

The electronic unit housing 17 and the mount unit 6 are rotatably linked by the hinge mechanism 58.

When the electronic unit housing 17 and the mount unit 6 are coupled (face to face), the electronic unit housing 17 is coupled so as to rotate with the hinge mechanism 58 as its fulcrum.

Here, the angle formed by the mount unit 6 and the electronic unit housing 17 around the hinge mechanism 58 is defined as the rotation angle.

FIG. 8a shows the state when the rotation angle of the electronic unit housing 17 and the mount unit 6 is about 90 degrees (non-attached state). FIG. 8b shows the state (non-attached state) when the rotation angle of the electronic unit housing 17 and the mount unit 6 is about 30 degrees. In these non-contact states, the electronic unit housing 17 and the mount unit 6 are attached to each other via the hinge mechanism 58, and the sensor connection terminal 7 and the second connection terminal 10 are not yet connected. This state is defined as the first state.

FIG. 8c shows the state (attached state) when the electronic unit housing 17 and the mount unit 6 have a rotation angle of zero degrees, and the electronic unit 3 and the mount unit 6 are completely coupled. This is a state in which the sensor connection terminal 7 and the second connection terminal 10 are connected. This state is defined as the second state.

Thus, the electronic unit housing 17 and the mount unit 6 are linked to each other by the hinge mechanism 58. The hinge mechanism 58 forms the first state in which the mount unit 6 and the electronic unit housing 17 (electronic unit 3) are attached to each other in a state where the sensor connection terminal 7 linked to the sensor 5 and the second connection terminal 10 provided on the electronic unit 3 side have yet to be connected.

This first state is a state in which the sensor connection terminal 7 and the second connection terminal 10 have yet to be connected to each other, as shown in FIGS. 8a and 8b.

Furthermore, the hinge mechanism 58 forms the second state in which the mount unit 6 and the electronic unit housing 17 (electronic unit 3) are mounted in a state where the sensor connection terminal 7 linked to the sensor 5 and the second connection terminal 10 provided on the electronic unit 3 side have been connected.

This second state is a state in which the sensor connection terminal 7 and the second connection terminal 10 are connected to each other, as shown in FIG. 8c.

Thus, in this embodiment, the hinge mechanism 58 forms a first state in which the mount unit 6 and the electronic unit housing 17 (electronic unit 3) are mounted to each other in a state where the sensor connection terminal 7 and the second connection terminal 10 have yet to be connected to each other, and a second state in which the mount unit 6 and the electronic unit housing 17 (electronic unit 3) are mounted to each other in a state where the sensor connection terminal 7 and the second connection terminal 10 have been connected. This makes it easy to attach the wearable sensor device 1 to the patient's body.

That is, after the sensor 5 is inserted into the body, the electronic unit 3 is mounted on the mount unit 6 by the simple operation of rotating the electronic unit housing 17 with respect to the mount unit 6 via the hinge mechanism 58 to go from the first state to the second state. Therefore, the wearable sensor device 1 can be easily attached to the patient's body.

Consequently, even if the place where the sensor is to be attached cannot be seen (such as the outside of the upper arm part 2), the electronic unit housing 17 can be mounted to the mount unit 6, and the sensor connection terminal 7 and the second connection terminal 10 can be connected, by the simple operation of rotating the electronic unit 3 via the hinge mechanism 8. As a result, the wearable sensor device 1 is easy to attach to the patient's body.

Since the mount unit 6 and the electronic unit 3 are rotatably linked together via the hinge mechanism 8, there is no need to grasp the relatively small electronic unit 3 to attach it to the mount unit and the sensor. As a result, it is easy to attach the wearable sensor device 1 to the body.

Further, the hinge mechanism 58 is provided with an elastic body as described above. The elastic body is configured to bias the electronic unit 3 in the direction of mounting to the mount unit 6.

In this embodiment, the elastic body is constituted by the spring unit 16 provided to the hinge mechanism 58. The spring unit 16 is formed integrally with the hinge portion 15 using a resin or other such material. Therefore, the hinge mechanism 58 having an elastic body can be formed without increasing the number of parts.

Consequently, as shown in FIG. 8b, the electronic unit housing 17 of the wearable sensor device 1 will be substantially closed with respect to the mount unit 6 even when not being manually operated by the user.

As a result, the user can create a state in which the electronic unit housing 17 is mounted on the mount unit 6 simply by lightly pressing the electronic unit housing 17 from above with a finger as shown in FIG. 8c. Here again, since a downward load is exerted on the wearable sensor device 1 from above, the bottom surface of the mount unit 6 is pressed against the skin to which the device is to be mounted. As a result, the wearable sensor device 1 is more securely attached to the skin of the upper arm 2.

Also, since the electronic unit housing 17 covers the sensor connection terminal 7 disposed on the mount unit 6 as shown in FIG. 8b, this prevents the user from accidentally touching a finger to the second connection terminal 10 provided on the bottom surface of the electronic unit 3 and the sensor connection terminal 7.

The elastic body may be a torsion spring provided to the hinge mechanism, or may be a leaf spring.

Furthermore, a through-hole 14 through which passes a needle for puncturing the skin and inserting the sensor 5 into the body is provided above the sensor 5 of the mount unit 6.

In this embodiment, the electronic unit housing 17 is detachably attached to the mount unit 6 via the hinge mechanism 8 serving as a mounting mechanism. The electronic unit 3 is removably attached to the electronic unit housing 17.

Consequently, the electronic unit 3 can be attached to the mount unit 6 after the electronic unit housing 17, the mount unit 6, and the sensor 5 attached to the mount unit 6 have been sterilized, which allows sterilization to be carried out in a state in which the electronic unit 3 has been separated.

Here, the reason for performing the sterilization with the electronic unit 3 separated is that the electronic unit 3 cannot be sterilized when electron beam sterilization is employed, for example, and therefore the electronic unit 3 must be separated from the member to be sterilized. Accordingly, because the electronic unit housing 17 is detachably mounted on the mount unit 6 via the hinge mechanism 8, sterilization can be performed with the electronic unit 3 in a separated state.

### Embodiment 4

The configuration of the sensor insertion device 25 according to yet another embodiment of the present invention will now be described.

With the sensor insertion device 25 of this embodiment, the form of a support unit 130 that supports the electronic unit 3 differs from the support unit 30 in the above embodiments. The rest of the configuration is the same as in the above embodiments, and components that are the same will be numbered the same and detailed description thereof will be omitted.

FIGS. 15a to 15d show the internal state of the sensor insertion device 25 at the time of sensor insertion.

As shown in FIG. 15a, the sensor insertion device 25 comprises, inside the main body case (the upper case 26 and the lower case 28), the sensor insertion mechanism 29 that inserts the sensor 5 into the body, the support unit 30 that supports the electronic unit 3 in the above-mentioned first state while the sensor 5 is being inserted into the body, and the holding unit 31 that holds the mount unit 6.

The sensor insertion mechanism 29 has a puncture needle 32, a puncture needle holder 33, and a drive unit 34 that drives the puncture needle holder 33 downward in the puncturing direction, and pulls up the puncture needle holder 33 after the puncture to withdraw the puncture needle 32 from the skin.

Just as in Embodiment 1, this sensor insertion mechanism 29 will not be described in order to avoid complicating the overall description.

The support unit 130 is provided as a side surface of the puncture needle holder 33 in the upper case 26, and in the state shown in FIGS. 15a to 15c, the electronic unit 3 provides support in a state in which the rotation angle is about 90 degrees with respect to the mount unit 6.

The description will now return to FIGS. 15a to 15d.

FIG. 15a shows a state in which the upper case 26 has yet to be pushed downward.

Inside the upper case 26, the mount unit 6 of the wearable sensor device 1 is held by the holding unit 31 in the lower part of the upper case 26. The puncture needle 32 is in a state in which the sensor 5 is included therein.

The electronic unit 3 connected to the mount unit 6 by the hinge mechanism 8 is in a state of being supported by the support unit 130.

The electronic unit 3 connected to the mount unit 6 by the hinge mechanism 8 is supported by the support unit 130. The electronic unit 3 is rotatably linked to the mount unit 6 by the hinge mechanism 8. Therefore, the support unit 130 only needs to support the electronic unit 3 so as not to rotate.

FIG. 15b shows a state in which the upper case 26 has been pushed downward. In this state, the puncture needle 32 has pierced the skin with the sensor 5 contained therein.

FIG. 15c shows a state in which the puncture needle holder 33 has been pulled up by the drive unit 34. In this state, the sensor 5 is separated from the puncture needle 32 and is left under the skin.

Here, the support unit 130 of the electronic unit 3 is fixed to the puncture needle holder 33, so when the puncture needle holder 33 is lifted up, the support unit 130 is also lifted up, and the support of the electronic unit 3 is released. That is, the electronic unit 3 is supported by the support unit 130 in the above-mentioned first state until the sensor 5 is inserted into the patient's body. Then, after the sensor 5 has been inserted into the body, the support unit 130 releases its support of the electronic unit 3.

FIG. 15d shows a state in which the user has lifted up the sensor insertion device 25.

Consequently, the mount unit 6 and the electronic unit 3 of the wearable sensor device 1 can form a first state of being mounted in a state where the sensor connection terminal 7 and the second connection terminal 10 have yet to be connected, and a second state of being mounted in a state where the connection terminal 7 and the second connection terminal 10 have been connected. As a result, the wearable sensor device 1 easily attached to the patient's body.

That is, in the configuration of this embodiment, after the sensor 5 is inserted into the body, the support of the electronic unit 3 by the support unit 130 is released, there is a change from the first state to the second state, and the electronic unit 3 is mounted on the mount unit 6. As a result, the wearable sensor device 1 can be easily attached to the patient's body.

In this embodiment, in addition to the configuration of the sensor insertion device 25 of the above-mentioned Embodiments 1 and 2, a configuration in which the electronic unit 3 and the mount unit 6 are rotatably mounted via the hinge mechanism 8 was also described as an example.

However, the present invention can be similarly applied to a configuration in which the electronic unit housing 17 and the mount unit 6 are rotatably mounted via the hinge mechanism 8. In this configuration, the support unit 130 is configured to support the electronic unit housing 17.

### INDUSTRIAL APPLICABILITY

The wearable sensor device of the present invention is very much expected to be applied to a blood glucose level sensor in a continuous blood glucose measurement system, for example.

### REFERENCE SIGNS LIST

1 wearable sensor device
2 upper arm
3 electronic unit
4 measurement device
5 sensor
6 mount unit
7 sensor connection terminal (first connection terminal)
8 hinge mechanism (mounting mechanism)
8a, 8b hinge member
9 insertion slot
10 second connection terminal
11 O-ring
12, 13 locking mechanism
12a, 12b snap fit
13a, 13b insertion opening
14 through-hole
15 hinge portion
16 spring portion (elastic body)
17 electronic unit housing
18 sensor unit
19 measurement unit
20 control unit
21 temperature sensor
22 storage unit
23 communication unit
24 battery
25 sensor insertion device
26 upper case
27 finger
28 lower case
29 sensor insertion mechanism
30 support unit
31 holding unit
32 puncture needle
33 puncture needle holder
34 drive unit
58 hinge mechanism (mounting mechanism)
130 support unit

## Claims

1. A wearable sensor device, comprising:
a sensor at least part of which is inserted into a human body, and which has a first connection terminal;
a mount unit on which at least part of the first connection terminal of the sensor is disposed and which is mounted on a skin of the human body;
an electronic unit that has a second connection terminal connected to the first connection terminal of the sensor, and a control unit configured to acquire signals from the sensor; and
a mounting mechanism configured to link the electronic unit to the mount unit in a rotatable state and mount the electronic unit to the mount unit, the mounting mechanism configured to form a first state where the mount unit and the electronic unit are mounted in a state in which the first connection terminal and the second connection terminal have yet to be connected, and a second state where the mount unit and the electronic unit are mounted in a state in which the first connection terminal and the second connection terminal have been connected.

2. The wearable sensor device according to claim 1,
wherein the mounting mechanism is a hinge mechanism.

3. The wearable sensor device according to claim 1or 2,
wherein the mounting mechanism has an elastic body configured to urge the electronic unit in a direction of mounting to the mount unit.

4. The wearable sensor device according to any of claims 1 to 3,
further comprising a locking mechanism configured to fix the electronic unit and the mount unit in a state in which the first connection terminal and the second connection terminal are connected.

5. The wearable sensor device according to any of claims 1 to 4,
wherein the mounting mechanism is configured to be attachable to and detachable from at least one of the electronic unit and the mount unit.

6. A wearable sensor device, comprising:
a sensor at least part of which is inserted into a human body, and which has a first connection terminal;
a mount unit on which at least the first connection terminal of the sensor is disposed and which is mounted on a skin of the human body;
an electronic unit housing;
a mounting mechanism configured to link the electronic unit to the mount unit in a rotatable state and mount the electronic unit housing to the mount unit; and
an electronic unit that is detachably provided in the electronic unit housing and has a second connection terminal that is connected to the first connection terminal, and a control unit configured to acquire signals from the sensor,
the mounting mechanism configured to form a first state where the mount unit and the electronic unit are mounted in a state in which the first connection terminal and the second connection terminal have yet to be connected, and a second state where the mount unit and the electronic unit are mounted in a state in which the first connection terminal and the second connection terminal have been connected.

7. The wearable sensor device according to claim 6,
wherein the mounting mechanism is a hinge mechanism.

8. The wearable sensor device according to claim 6 or 7,
wherein the mounting mechanism has an elastic body configured to urge the electronic unit housing in a direction of mounting to the mount unit.

9. The wearable sensor device according to any of claims 6 to 8,
further comprising a locking mechanism configured to fix the electronic unit and the mount unit in a state in which the first connection terminal and the second connection terminal are connected.

10. The wearable sensor device according to any of claims 6 to 9,
wherein a battery configured to supply power to the electronic unit is disposed in the electronic unit housing.

11. A sensor insertion device configured to insert into a human body a sensor of a wearable sensor device, the wearable sensor device comprising:
a sensor at least part of which is inserted into the human body and which has a first connection terminal;
a mount unit on which at least part of the first connection terminal of the sensor is disposed and which is mounted on a skin of the human body;
an electronic unit that has a second connection terminal connected to the first connection terminal of the sensor, and a control unit configured to calculate biometric information from signals inputted from the sensor; and
a mounting mechanism configured to link the electronic unit to the mount unit in a rotatable state and mount the electronic unit to the mount unit, the mounting mechanism configured to form a first state where the mount unit and the electronic unit are mounted in a state in which the first connection terminal and the second connection terminal have yet to be connected, and a second state where the mount unit and the electronic unit are mounted in a state in which the first connection terminal and the second connection terminal have been connected,
the sensor insertion device comprising:
a main body case to which the wearable sensor device is attached;
a sensor insertion mechanism that is provided to the main body case and inserts the sensor into the human body; and
a support unit configured to support the electronic unit in the first state in a part of the main body case until the sensor is inserted into the human body by the sensor insertion mechanism.

12. The sensor insertion device according to claim 11,
further comprising a holding portion that is provided to the main body case and configured to hold the mount unit in the first state until the sensor insertion mechanism inserts the sensor.

13. The sensor insertion device according to claim 11 or 12,
wherein the mounting mechanism is a hinge mechanism.

14. The sensor insertion device according to any of claims 11 to 13,
wherein the mounting mechanism has an elastic body configured to urge the electronic unit in a direction of mounting to the mount unit.

15. The sensor insertion device according to any of claims 11 to 14,
further comprising a locking mechanism configured to fix the electronic unit and the mount unit in a state in which the first connection terminal and the second connection terminal are connected.

16. The sensor insertion device according to any of claims 11 to 15,
wherein the mounting mechanism detachably links the electronic unit and the mount unit.

17. The sensor insertion device according to any of claims 11 to 16,
further comprising an electronic unit housing in which the electronic unit is detachably installed,
wherein the electronic unit housing is mounted to the mount unit by the mounting mechanism.

18. A sensor insertion method for inserting into a human body a sensor of a wearable sensor device, the wearable sensor device comprising:
a sensor at least part of which is inserted into the human body and which has a first connection terminal;
a mount unit on which at least part of the first connection terminal of the sensor is disposed and which is mounted on a skin of the human body;
a mounting mechanism configured to link the electronic unit to the mount unit in a rotatable state and mount an electronic unit to the mount unit; and
an electronic unit that is mounted to the mount unit via the mounting mechanism, has a second connection terminal connected to the first connection terminal of the sensor, and has a control unit configured to calculate biometric information from signals inputted from the sensor,
wherein the mounting mechanism configured to form a first state where the mount unit and the electronic unit are mounted in a state in which the first connection terminal and the second connection terminal have yet to be connected, and a second state where the mount unit and the electronic unit are mounted in a state in which the first connection terminal and the second connection terminal have been connected,
the sensor insertion method comprising the steps of:
disposing the mount unit on the skin in the first state in which the first connection terminal of the mount unit and the second connection terminal of the electronic unit have yet to be connected;
inserting into the human body the sensor mounted to the mount unit; and
connecting the first connection terminal of the mount unit and the second connection terminal of the electronic unit, resulting in the second state, after the sensor has been inserted into the human body.

19. The sensor insertion method according to claim 18,
wherein the mounting mechanism is a hinge mechanism.

20. The sensor insertion method according to claim 18 or 19,
wherein the mounting mechanism has an elastic body that urges the electronic unit in a direction of mounting to the mount unit.

21. The sensor insertion method according to any of claims 18 to 20,
further comprising a locking mechanism configured to fix the electronic unit and the mount unit in the second state in which the first connection terminal and the second connection terminal are connected.

22. The sensor insertion method according to any of claims 18 to 21,
wherein the mounting mechanism is detachably provided to the electronic unit and/or the mount unit.
